# EUROPEAN PATENT SPECIFICATION

(11) **EP 1 738 755 B1**
(45) Date of publication and mention of the grant of the patent: **30.05.2018**
(21) Application number: 05730445.3
(22) Date of filing: 14.04.2005
(51) Int. Cl.: A61K 31/198, A61K 9/20, A61K 47/26, A61K 47/38, A61P 1/16, A61P 3/10, A61P 9/10, A61P 13/12, A61P 21/06, A61P 25/20, A61K 47/18

(54) **TABLET CONTAINING BRANCHED CHAIN AMINO ACID AND PROCESS FOR PRODUCING THE SAME**
TABLETTE ENTHALTEND VERZWEIGTKETTIGE AMINOSÄURE UND HERSTELLUNGSVERFAHREN DAFÜR
COMPRIME CONTENANT UN ACIDE AMINE A CHAÎNE RAMIFIEE ET PROCESSUS DE FABRICATION DE CELUI-CI

(30) Priority: 14.04.2004 JP 2004118751
(43) Date of publication of application: 03.01.2007
(73) Proprietor: Kyowa Hakko Bio Co., Ltd., Tokyo, 100-8185 (JP)
(72) Inventor: HARA, Takahiro, Tokyo 1008185 (JP); KIMURA, Masao, Tokyo 1008185 (JP); SAKAI, Yasushi, Tokyo 1008185 (JP)
(74) Representative: Vossius & Partner Patentanwälte Rechtsanwälte mbB
(86) International application number: PCT/JP2005/007203
(87) International publication number: WO 2005/099681

(56) References cited:
- EP-A1- 0 354 442
- WO-A1-01/06872
- JP-A- 8 256 695
- JP-A- 2000 197 454
- JP-A- 2000 197 454
- JP-A- 2001 089 395
- JP-A- 2001 089 395
- JP-A- 2001 258 509
- JP-A- 2003 221 326
- US-A- 5 994 324
- US-A1- 2004 047 904

## Description

### Technical Field

The present invention relates to a tablet comprising a branched chain amino acid and a method for producing the same.

### Background Art

It is considered that valine, leucine, isoleucine and the like, which are known as branched chain amino acids, are effective for suppressing degradation of muscular proteins, preventing ischemic heart failure, protecting the kidney, improving sleep disorder in patients with renal failure, improving diabetes mellitus and the like. Pharmaceutical preparations comprising valine, leucine and isoleucine as active ingredients are known as drug products for treating hepatic diseases. Further, in recent years, nutritional physiological functions of branched chain amino acids have attracted attention, imparting high market value to drinks, food products and the like comprising these amino acids. Intake forms of branched chain amino acids include drinks, granules, tablets and the like. However, since branched chain amino acids exhibit characteristic bitterness, it has been needed to contrive the taste in such drinks, granules, tablets and the like. In addition, when tablets comprising a branched chain amino acid are tried to be produced, there frequently happen compression molding failures such as adhesion of the branched chain amino acids to punches and dies of a compression molding machine, capping and sticking, thereby making preparation of tablets difficult. For this reason, it has been needed to prevent such compression molding failures.

Heretofore, several methods for preventing compression molding failures in producing tablets comprising a branched chain amino acid have been disclosed (for example, Patent Documents 1 to 3). Patent Document 1 describes that a tablet comprising a branched chain amino acid can be produced without compression molding failures by using lactitol as a diluent. In this method, powdery raw material comprising 0.12 to 2 parts by mass of lactitol and 1 part by mass of a branched chain amino acid was granulated and then dried to prepare a granule, the granule was mixed with microcrystalline cellulose as a diluent and vegetable oil powder as a lubricant, and then the resultant mixture was compression molded. Meanwhile, in the method described in Patent Document 2, a mixture comprising an amino acid such as a branched chain amino acid, one compound selected from the group consisting of lactose, maltose, trehalose, sorbitol, lactitol, mannitol, xylitol and maltitol, and a disintegrant was granulated using an aqueous solution of a binder and dried to prepare a granule and the obtained granule is compression molded to produce an orally disintegrating tablet comprising an amino acid. Further, Patent Document 3 relates to a chewable preparation comprising branched chain amino acids and describes that a particulate mixture comprising three kinds of branched chain amino acids, that is, isoleucine, leucine and valine, as active ingredients was granulated to prepare a granule, said granule was coated with a coating liquid comprising a binder, and said coated granule was compression molded.

On the other hand, hydrogenated palatinose is a sweetener made from sucrose as a raw material. Hydrogenated palatinose does not cause tooth decay and is suitable to obese persons and patients with diabetes mellitus because of its low calorie value and no contribution to blood glucose increase. Furthermore, hydrogenated palatinose is chemically stable, does not exhibit decomposition coloring with heat, an acid or an alkali, dose not undergo Maillard reaction and has low hygroscopicity, exhibiting excellent properties in compatibility with food product and the like. It is known that granulated hydrogenated palatinose is suitable to compression molding (for example, Patent Document 4).
Patent Document 1: Japanese Published Unexamined Patent Application No. 2001-258509
Patent Document 2: Pamphlet of WO 03/041698
Patent Document 3: Japanese Published Unexamined Patent Application No. 2003-221326
Patent Document 4: Japanese Published Unexamined Patent Application No. 2000-197454
US2004/0047904 aims at providing amino acid-containing tablets and methods for producing the same. It discloses intraorally disintegrable tablets comprising amino acids such as valine, leucine and isoleucine combined with a sweetening agent and a binding agent, which may be a saccharide, such as lactose.

### Disclosure of the Invention

### Problems to be Solved by the Invention

An object of the present invention is to provide a tablet comprising a branched chain amino acid that can be produced without compression molding failures and a method for producing the same.

### Means for Solving the Problems

The present invention relates to the following embodiments (1) to (13):
(1) A tablet comprising 30 to 80 % by mass of a branched chain amino acid and hydrogenated palatinose;
(2) The tablet according to the above (1), wherein the tablet has a tablet hardness of 20 to 200 N.
(3) The tablet according to the above (1) or (2), wherein the tablet is produced using hydrogenated palatinose having a volume average particle diameter of 0.5 to 400 µm.
(4) The tablet according to any of the above (1) to (3), wherein the tablet further comprises cellulose.
(5) The tablet according to the above (4), wherein the cellulose is microcrystalline cellulose and the tablet is produced using microcrystalline cellulose having a volume average particle diameter of 20 to 100 µm.
(6) The tablet according to any the above (1) to (5), wherein the branched chain amino acid is valine.
(7) The tablet according to any of the above (1) to (5), wherein the branched chain amino acid is leucine.
(8) The tablet according to any of the above (1) to (5), wherein the branched chain amino acid is isoleucine.
(9) The tablet according to any of the above (1) to (5), wherein the branched chain amino acid is a mixture of valine, leucine and isoleucine.
(10) The tablet according to the above (9), wherein the weight ratio of valine, leucine and isoleucine is 1 : (0.6 to 6) : (0.3 to 3).
(11) The tablet according to any of the above (1) to (10), wherein the tablet comprises 0 to 20 % by mass lubricant.
(12) A method for producing a tablet comprising a branched chain amino acid and hydrogenated palatinose as defined in any one of the above (1) to (11), which comprises compression molding a mixture comprising the branched chain amino acid and hydrogenated palatinose by direct tableting method.
(13) A method for producing a tablet comprising a branched chain amino acid and hydrogenated palatinose as defined in any one of the above (1) to (11), which comprises granulating a mixture comprising the branched chain amino acid and hydrogenated palatinose and compression molding the resulting granules.

### Effect of the Invention

According to the present invention, a tablet comprising a branched chain amino acid that can be produced without compression molding failures and a method for producing the same are provided.

### Best Mode for Carrying Out the Invention

The tablet of the present invention comprises a branched chain amino acid and hydrogenated palatinose, preferably further comprises cellulose and, if desired, optionally comprises sugars, lubricants, sweeteners, acidulants, binders, antioxidants, color agents, flavors, diluents, disintegrants or the like.

The branched chain amino acid in the present invention includes, for example, valine, leucine, isoleucine, a mixture of two or three kinds of them, and the like. There are no particular limitations on the method for producing the branched chain amino acid, and it may be produced by fermentation method, synthetic method, purification method and the like. The particle size distribution of the branched chain amino acid is not particularly limited. The branched chain amino acid may be purchased from, for example, Kyowa Hakko Kogyo Co., Ltd. or Kyowa Wellness Co., Ltd. The content of the branched chain amino acid in the tablet of the present invention is preferably 10 to 80% by mass, more preferably 30 to 60% by mass. The branched chain amino acid is preferably one of valine, leucine and isoleucine, or a mixture of valine, leucine and isoleucine. In said mixture, the weight ratio of each component is preferably 1 : (0.6 to 6) : (0.3 to 3) and more preferably 1 : (1 to 3) : (0.5 to 1.5). Here, each of valine, leucine and isoleucine may be any of L-form, D-form and a mixture of L-form and D-form, and is preferably L-form.

The hydrogenated palatinose in the present invention includes, for example, α-D-glucopyranosyl-1,6-sorbitol (GPS), α-D-glucopyranosyl-1,6-mannitol (GPM) and a mixture thereof. When the hydrogenated palatinose is a mixture of GPS and GPM, although the mixed ratio of GPS and GPM is not particularly limited, an equimolar mixture is preferred. The method for producing the hydrogenated palatinose is not particularly limited. For example, hydrogenated palatinose can be produced by treating sugar with sugar transferase to produce palatinose and subsequently reducing it. In producing the tablet of the present invention, the particle diameter of the hydrogenated palatinose used is preferably 0.5 to 400 *µ*m, more preferably 5 to 200 *µ*m, as the volume average particle diameter determined by microscopic method or sieving method. Hydrogenated palatinose may be purchased from, for example, Mitsui Sugar Co., Ltd. (Product name: Palatinit).

The cellulose in the tablet of the present invention includes, for example, microcrystalline cellulose. In producing the tablet of the present invention, the particle diameter of the microcrystalline cellulose used is, although not limited to a particular range, preferably approximately 20 to 100 *µ*m, more preferably approximately 30 to 60 *µ*m, as the volume average particle diameter determined by microscopic method or sieving method. The particles of the microcrystalline cellulose are preferably irregular rod shape particles having micropores in order to enhance hardness, disintegratability and other properties of the tablet. In addition, the particles of the microcrystalline cellulose are preferably excellent in fluidity in order to enhance homogeneity in content, production speed of the tablet and the like. There are no particular limitations on the method for producing the microcrystalline cellulose. It can be produced, for example, by a method in which pulp fiber or the like is hydrolyzed, non-microcrystalline cellulose is removed from the hydrolyzed product, and the resultant material is pulverized and dried. Microcrystalline cellulose may be purchased from, for example, Asahi Kasei Corporation (Product name: Avicel). The content of cellulose in the tablet of the present invention is not particularly limited as long as it is within a range generally used in formulation, and it is preferably 0 to 30% by mass.

The saccharide, which is not particularly limited as long as it is usable for food product or the like, includes, for example, monosaccharides, disaccharides, sugar alcohols, oligosaccharides and the like.

The monosaccharides include, for example, glucose, xylose, galactose, fructose and the like. The disaccharides include, for example, trehalose, sucrose, lactose, palatinose and the like. The sugar alcohols include, for example, maltitol, erythritol, sorbitol, xylitol and the like. The oligosaccharides include, for example, raffinose, inulooligosaccharide (chicory oligosaccharide), palatinose oligosaccharide and the like.

The form of the saccharides used in producing the tablet of the present invention, which is not particularly limited, is preferably a microcrystalline or microparticulate form. The particle diameter is, for example, 1 to 100 *µ*m, preferably 5 to 80 *µ*m, more preferably 10 to 60 *µ*m, particularly preferably 30 to 50 *µ*m, as the volume average particle diameter determined by microscopic or sieving method. The content of the saccharide in the tablet of the present invention is not particularly limited as long as it is within a range generally used in formulation.

The lubricant, which is not particularly limited as long as it is usable for food product or the like, includes, for example, stearic acid or metal salts thereof such as stearic acid, magnesium stearate and calcium stearate, sucrose esters of fatty acids or glycerol esters of fatty acids, hydrogenated oil and fat and the like. The lubricants may be present only on the surface of the tablet or dispersed in the tablet. The content of the lubricants in the tablet of the present invention is preferably 0 to 20% by mass, more preferably 0 to 5% by mass, further more preferably 0 to 1% by mass, most preferably 0%. The tablet of the present invention can be produced without compression molding failures even if the lubricant is not substantially contained, and also from the viewpoints of disintegrating property of the tablet after ingestion and stability of the ingredients of the tablet, it is preferred to use substantially no lubricant.

The sweetener, which is not particularly limited as long as it is usable for food product or the like, includes, for example, saccharin sodium, dipotassium glycyrrhizinate, aspartame, stevia, thaumatin and the like. The content of the sweetener in the tablet of the present invention is not particularly limited as long as it is within a range generally used in formulation.

The acidulant, which is not particularly limited as long as it is usable for food product or the like, includes, for example, citric acid, tartaric acid, malic acid and the like. The content of the acidulants in the tablet of the present invention is not particularly limited as long as it is within a range generally used in formulation.

The binder, which is not particularly limited as long as it is usable for food product or the like, includes, for example, gelatin, pullulan and the like. The content of the binder in the tablet of the present invention is not particularly limited as long as it is within a range generally used in formulation.

The antioxidant, which is not particularly limited as long as it is usable for food product or the like, includes, for example, tocopherol, ascorbic acid, cystein hydrochloride, stearate ester of L-ascorbic acid and the like. The content of the antioxidant in the tablet of the present invention is not particularly limited as long as it is within a range generally used in formulation.

The color agent, which is not particularly limited as long as it is usable for food product or the like, includes, for example, food yellow No. 5, food red No. 2, food blue No. 2, carotenoid pigments, tomato pigments and the like. The content of the color agent in the tablet of the present invention is not particularly limited as long as it is within a range generally used in formulation.

The flavor, which is not particularly limited as long as it is usable for food product or the like, includes, for example, lemon flavor, lemon lime flavor, grapefruit flavor, apple flavor and the like. The content of the flavor in the tablet of the present invention is not particularly limited as long as it is within a range generally used in formulation.

The diluent, which is not particularly limited as long as it is usable for food product or the like, includes, for example, hydroxypropylcellulose with a low degree of substitution and the like. The content of the diluent in the tablet of the present invention is not particularly limited as long as it is within a range generally used in formulation.

The disintegrant, which is not particularly limited as long as it is usable for food product or the like, includes, for example, corn starch, potato starch and the like. The content of the disintegrant in the tablet of the present invention is not particularly limited as long as it is within a range generally used in formulation.

The hardness of the tablet of the present invention is preferably high enough to avoid for example, cracking, crumbling and the like. The value of tablet hardness, which is generally measured with a tablet hardness tester as the force needed to crush the tablet in its radial direction, is preferably 20 to 200 N, more preferably 30 to 150 N, particularly preferably 40 to 100 N. The tablet hardness can be measured with a commercial tablet hardness tester, for example, TH-203CP manufactured by Toyama Sangyo Co., Ltd.

The shape of the tablet of the present invention is, although not particularly limited, preferably, for example, round shape, triangular shape, shot-like shape or the like. The size of tablet of the present invention is not particularly limited. Preferably the tablet has, for example, a mass of 0.1 to 2 g, and a diameter of 0.3 to 2.0 cm.

The tablet of the present invention can be produced, for example, by a production method comprising a step in which (i) the above-described ingredients of said tablet, which are in powdery state, are mixed as they are, (ii) a part of said ingredients are granulated and then the granule is mixed with the remaining ingredients or (iii) all of said ingredients are granulated; and then a step in which the mixture or granule obtained is compression molded to produce the tablet. More specifically, by a production method in which the ingredients of said tablet comprising a branched chain amino acid and hydrogenated palatinose are mixed and the resultant mixture is compression molded by direct tableting method, the tablet having the desired hardness can be produced without compression molding failures. Alternatively by a production method in which, among the ingredients of said tablet comprising a branched chain amino acid and hydrogenated palatinose, a mixture of the branched chain amino acid and hydrogenated palatinose is granulated and then a mixture of said ingredients is compression molded (production method involving compression molding by indirect tableting method), the tablet having the desired hardness can be also produced without compression molding failures. The purity of each branched chain amino acid is preferably 95% or more, more preferably 98% or more. The water content of the branched chain amino acid is preferably 1% or less, more preferably 0.5% or less. The apparatus used in compression molding is not particularly limited and for example, a press such as a rotary press and a hydraulic press may be used. The aforementioned method involving compression molding by direct tableting method is preferred because it is quite a simple method in which the ingredients of the tablet are only mixed and then compression molded. This method is also preferred in terms of stability and the like of the ingredients of the tablet because the production steps involve no addition of water or the like to said ingredients.

By the method for producing a tablet of the present invention, both in the case wherein a lubricant is mixed and in the case wherein no lubricant is used substantially, the tablet having the desired hardness can be obtained from each of the aforementioned mixtures or the aforementioned granule without compression molding failures. Considering this point, as well as from the viewpoints of disintegrating property of the tablet after ingestion and stability of the ingredients of the tablet, it is preferred to use substantially no lubricant. Moreover, by using so-called external lubricating compression molding method, in which punches and dies of a compression molding apparatus are coated in advance with quite a small amount of a lubricant and a mixture comprising no lubricant is compression molded using the compression molding apparatus having punches and dies coated with a lubricant, the tablet having the desired hardness can be produced without compression molding failures. This method is also preferred in terms of the disintegrating property after ingestion of the tablet, the stability of the ingredients of the tablet and the like, as in the case of the above-described method, because only quite a small amount of a lubricant is used.

When all or a part of the ingredients of the tablet of the present invention are granulated (for example, in the aforementioned method involving compression molding by indirect tableting method), the granulation method includes, for example, wet granulating method using water, ethanol or the like, dry granulation method and the like. The apparatus used for granulation is not particularly limited, and there may be used, for example, a fluidized bed granulator, a rotary stirring granulator, an extruding granulator and the like. The aforementioned production method involving compression molding by indirect tableting method is preferred for making the contents of a branched chain amino acid and hydrogenated palatinose uniform. In granulation, it is preferred to reduce the addition of water or the like as much as possible in terms of the stability and the like of the ingredients of the tablet. By the aforementioned method of compression molding by indirect tableting method, even if addition of water or the like is reduced, a tablet having a desired hardness can be produced without compression molding failures.

In the following, the present invention will be described in more detail in reference to Examples, but the present invention is not restricted by these examples.

### Example 1

Tablets with the compositions listed in Table 1 were produced in accordance with the following procedures.

Branched chain amino acids (BCAA) (a mixture of 10 parts of L-valine, 20 parts of L-leucine and 10 parts of L-isoleucine, wherein L-valine and L-isoleucine were used after pulverized with a Model M-2 mill manufactured by Nara Machinery Co., Ltd. equipped with a 0.5 mm-screen), one compound selected from hydrogenated palatinose (Palatinit PNM-2 (Mitsui Sugar Co., Ltd.)), lactitol (Lactitol LC-1 (Nikken Fine Chemical Co., Ltd.)), maltitol (Mabit 50M (Hayashibara Shoji, Inc.)) and lactose (SUPER-TAB (Asahi Kasei Corporation)), and microcrystalline cellulose (Avicel FD101 (Asahi Kasei Corporation)) were thoroughly mixed in a polyethylene bag with blending ratios varied as listed in Table 1, and the resultant mixtures were compression molded with a one-shot compression molding apparatus (6B-2M vertical press manufactured by Kikusui Seisakusho Ltd.) to produce tablets having a diameter of 8 mm and a mass of 240 mg. Here, the compression force was adjusted so that the tablet hardness of 59 N (measured with a hardness meter KHT-20N manufactured by Fujiwara Scientific Co., Ltd.) was obtained in all the cases.

**[Table 1]**

| | | | | | |
|---|---|---|---|---|---|
| Composition | BCAA | 40 parts | 40 parts | 40 parts | 40 parts |
| | Hydrogenated palatinose | 40 parts | - | - | - |
| | Lactitol | - | 40 parts | - | - |
| | Maltitol | - | - | 40 parts | - |
| | Lactose | - | - | - | 40 parts |
| | Microcrystalline cellulose | 20 parts | 20 parts | 20 parts | 20 parts |
| Results of production | Hardness (fixed) | 59 N | 59 N | 59 N | 59 N |
| | Compression force | 13 kN | 16 kN | 7.4 to 8 kN | 12 kN |
| | Compression molding failures | None | Capping | Sticking | Sticking |

As shown in Table 1, when hydrogenated palatinose was used, the tablet having the desired hardness was produced without compression molding failures. When maltitol or lactitose was used, although the tablet having the desired hardness was produced, sticking took place. When lactitol was used, although the tablet having the desired hardness was produced, capping took place. That is, the tablet of the present invention comprising a branched chain amino acid and hydrogenated palatinose was produced as a tablet having the desired hardness without compression molding failures, under the condition that substantially no lubricant was contained, by the simple production method, in which a mixture of the ingredients of the tablet was compression molded by direct tableting method.

### Example 2

A mixture was prepared by blending 1360 g of L-leucine, 2240 g of hydrogenated palatinose and 400 g of microcrystalline cellulose. The mixture was compression molded using a compression molding apparatus with a compression force of 13 kN to produce tablets having a diameter of 8 mm and a mass of 240 mg. No compression molding failures were observed. The tablet hardness was 75 N (mean value with n = 20).

As shown in Example 2, the tablet of the present invention comprising a branched chain amino acid and hydrogenated palatinose was produced as a tablet having the desired hardness without compression molding failures, under the condition that substantially no lubricant was contained, by the simple production method, in which a mixture of the ingredients of the tablet was compression molded by direct tableting method.

### Example 3

A mixture of 1530 g of L-valine (pulverized in advance with a Model M-2 mill manufactured by Nara Machinery Co., Ltd. equipped with a 0.5 mm-screen; hereinafter L-valine was used after the same treatment as described here) and 1584 g of hydrogenated palatinose was granulated using 33.75 g pullulan (5% aqueous solution) with a fluidized bed granulator (FLO-5 manufactured by Freund Corporation). To 2798 g of the granule obtained, 1200 g of microcrystalline cellulose and 2 g of fine particles of silicon dioxide were added and mixed. The resultant mixture was compression molded using a compression molding apparatus (rotary press VIRGO524SS1AY manufactured by Kikusui Seisakusho Ltd.; hereinafter the same one was used) with a compression force of 12 kN to produce tablets having a diameter of 8 mm and a mass of 240 mg. No compression molding failures were observed. The tablet hardness was 83 N (mean value with n = 20).

### Example 4

A mixture of 1530 g of L-isoleucine (pulverized in advance with a Type M-2 mill manufactured by Nara Machinery Co., Ltd. equipped with a 0.5 mm-screen; hereinafter L-isoleucine was used after the same treatment as described here) and 1584 g of hydrogenated palatinose was granulated using 33.75 g pullulan (5% aqueous solution) with a fluidized bed granulator (FLO-5 manufactured by Freund Corporation). To 2798 g of the granule obtained, 1200 g of microcrystalline cellulose and 2 g of fine particles of silicon dioxide were added and mixed. The resultant mixture was compression molded using the compression molding apparatus with a compression force of 14 kN to produce tablets having a diameter of 8 mm and a mass of 240 mg. No compression molding failures were observed. The tablet hardness was 75 N (mean value with n = 20).

### Example 5

A mixture of 382.5 g of L-valine, 765 g of L-leucine, 382.5 g of L-isoleucine and 2038.5 g of hydrogenated palatinose was granulated using 29.25 g pullulan (5% aqueous solution) with a fluidized bed granulator (FLO-5 manufactured by Freund Corporation). To 3200 g of the granule obtained, 1200 g of microcrystalline cellulose was added and mixed. The resultant mixture was compression molded using the compression molding apparatus with a compression force of 18 kN to produce tablets having a diameter of 8 mm and a mass of 240 mg. No compression molding failures were observed. The tablet hardness was 72 N (mean value with n = 20).

### Example 6

A mixture of 8.5 kg of L-valine, 17 kg of L-leucine, 8.5 kg of L-isoleucine and 45.3 kg of hydrogenated palatinose was granulated using 0.7 kg of pullulan (5% aqueous solution) with a fluidized bed granulator (FLO-120 manufactured by Freund Corporation). To 77.6 kg of the granule obtained, 19.4 kg of microcrystalline cellulose was added and mixed using a conical blender (CB-1200 blender manufactured by Nippon Kansouki Co., Ltd.). The resultant mixture was compression molded using the compression molding apparatus with a compression force of 18 kN to produce tablets having a diameter of 8 mm and a mass of 240 mg. No compression molding failures were observed. The tablet hardness was 75 N (mean value with n = 20).

As shown in Examples 3 to 6, the tablet of the present invention comprising a branched chain amino acid and hydrogenated palatinose was produced, by the production method comprising a step of granulating a mixture comprising the branched chain amino acid and hydrogenated palatinose among the ingredients of the tablet and then a step of compression molding, as a tablet having the desired hardness without compression molding failures even if comprising substantially no lubricant.

### Industrial Applicability

The present invention provides a tablet comprising a branched chain amino acid that can be produced without compression molding failures and a method for producing the same.

## Claims

1. A tablet comprising 30 to 80 % by mass of a branched chain amino acid and hydrogenated palatinose.

2. The tablet according to claim 1, wherein the tablet has a tablet hardness of 20 to 200 N.

3. The tablet according to claim 1 or 2, wherein the tablet is produced using hydrogenated palatinose having a volume average particle diameter of 0.5 to 400 µm.

4. The tablet according to any of claims 1 to 3, wherein the tablet further comprises cellulose.

5. The tablet according to claim 4, wherein the cellulose is microcrystalline cellulose and the tablet is produced using microcrystalline cellulose having a volume average particle diameter of 20 to 100 µm.

6. The tablet according to any of claims 1 to 5, wherein the branched chain amino acid is valine.

7. The tablet according to any of claims 1 to 5, wherein the branched chain amino acid is leucine.

8. The tablet according to any of claims 1 to 5, wherein the branched chain amino acid is isoleucine.

9. The tablet according to any of claims 1 to 5, wherein the branched chain amino acid is a mixture of valine, leucine and isoleucine.

10. The tablet according to claim 9, wherein the weight ratio of valine, leucine and isoleucine is 1 : (0.6 to 6) : (0.3 to 3).

11. The tablet according to any of claims 1 to 10, wherein the tablet comprises 0 to 20 % by mass lubricant.

12. A method for producing a tablet comprising a branched chain amino acid and hydrogenated palatinose as defined in any one of claims 1 to 11, which comprises compression molding a mixture comprising the branched chain amino acid and hydrogenated palatinose by direct tableting method.

13. A method for producing a tablet comprising a branched chain amino acid and hydrogenated palatinose as defined in any one of claims 1 to 11, which comprises granulating a mixture comprising the branched chain amino acid and hydrogenated palatinose and compression molding the resulting granules.

## Patentansprüche

1. Eine Tablette, umfassend 30 bis 80 Massen-% einer verzweigtkettigen Aminosäure und hydrierte Palatinose.

2. Die Tablette nach Anspruch 1, wobei die Tablette eine Tablettenhärte von 20 bis 200 N aufweist.

3. Die Tablette nach Anspruch 1 oder 2, wobei die Tablette unter Verwendung von hydrierter Palatinose mit einem Volumenmittel des Teilchendurchmessers von 0,5 bis 400 µm hergestellt wird.

4. Die Tablette nach einem der Ansprüche 1 bis 3, wobei die Tablette ferner Cellulose umfasst.

5. Die Tablette nach Anspruch 4, wobei die Cellulose mikrokristalline Cellulose ist und die Tablette unter Verwendung von mikrokristalliner Cellulose mit einem Volumenmittel des Teilchendurchmessers von 20 bis 100 µm hergestellt wird.

6. Die Tablette nach einem der Ansprüche 1 bis 5, wobei die verzweigtkettige Aminosäure Valin ist.

7. Die Tablette nach einem der Ansprüche 1 bis 5, wobei die verzweigtkettige Aminosäure Leucin ist.

8. Die Tablette nach einem der Ansprüche 1 bis 5, wobei die verzweigtkettige Aminosäure Isoleucin ist.

9. Die Tablette nach einem der Ansprüche 1 bis 5, wobei die verzweigtkettige Aminosäure ein Gemisch von Valin, Leucin und Isoleucin ist.

10. Die Tablette nach Anspruch 9, wobei das Gewichtsverhältnis von Valin, Leucin und Isoleucin 1 : (0,6 bis 6) : (0,3 bis 3) beträgt.

11. Die Tablette nach einem der Ansprüche 1 bis 10, wobei die Tablette 0 bis 20 Massen-% Gleitmittel umfasst.

12. Ein Verfahren zur Herstellung einer Tablette, umfassend eine verzweigtkettige Aminosäure und hydrierte Palatinose wie in einem der Ansprüche 1 bis 11 definiert, welches Formpressen eines Gemischs, das die verzweigtkettige Aminosäure und hydrierte Palatinose umfasst, durch ein direktes Tablettierungsverfahren umfasst.

13. Ein Verfahren zur Herstellung einer Tablette, umfassend eine verzweigtkettige Aminosäure und hydrierte Palatinose wie in einem der Ansprüche 1 bis 11 definiert, welches Granulieren eines Gemischs, das die verzweigtkettige Aminosäure und hydrierte Palatinose umfasst, und Formpressen des resultierenden Granulats umfasst.

## Revendications

1. Comprimé comprenant 30 à 80 % en masse d'un acide aminé à chaîne ramifiée et du palatinose hydrogéné.

2. Comprimé selon la revendication 1, dans lequel le comprimé a une dureté de comprimé de 20 à 200 N.

3. Comprimé selon la revendication 1 ou 2, dans lequel le comprimé est produit par utilisation de palatinose hydrogéné ayant un diamètre des particules du volume moyen de 0,5 à 400 µm.

4. Comprimé selon l'une quelconque des revendications 1 à 3, dans lequel le comprimé comprend en outre de la cellulose.

5. Comprimé selon la revendication 4, dans lequel la cellulose est une cellulose microcristalline et le comprimé est produit par utilisation d'une cellulose microcristalline ayant un diamètre des particules du volume moyen de 20 à 100 µm.

6. Comprimé selon l'une quelconque des revendications 1 à 5, dans lequel l'acide aminé à chaîne ramifiée est la valine.

7. Comprimé selon l'une quelconque des revendications 1 à 5, dans lequel l'acide aminé à chaîne ramifiée est la leucine.

8. Comprimé selon l'une quelconque des revendications 1 à 5, dans lequel l'acide aminé à chaîne ramifiée est l'isoleucine.

9. Comprimé selon l'une quelconque des revendications 1 à 5, dans lequel l'acide aminé à chaîne ramifiée est un mélange de valine, de leucine et d'isoleucine.

10. Comprimé selon la revendication 9, dans lequel le rapport en poids de la valine, de la leucine et de l'isoleucine est de 1 / (0,6 à 6) / (0,3 à 3).

11. Comprimé selon l'une quelconque des revendications 1 à 10, dans lequel le comprimé comprend 0 à 20 % en masse d'un lubrifiant.

12. Méthode de production d'un comprimé comprenant un acide aminé à chaîne ramifiée et du palatinose hydrogéné tel que défini dans l'une quelconque des revendications 1 à 11, qui comprend le moulage par compression d'un mélange comprenant l'acide aminé à chaîne ramifiée et le palatinose hydrogéné par une méthode de fabrication de comprimés par compression directe.

13. Méthode de production d'un comprimé comprenant un acide aminé à chaîne ramifiée et du palatinose hydrogéné tel que défini dans l'une quelconque des revendications 1 à 11, qui comprend la granulation d'un mélange comprenant l'acide aminé à chaîne ramifiée et le palatinose hydrogéné et le moulage par compression des granules résultants.
